(19) Europäisches Patentamt
European Patent Office
Office européen des brevets

(11) EP 2 146 199 A1

(12) EUROPEAN PATENT APPLICATION

(43) Date of publication:
20.01.2010 Bulletin 2010/03

(51) Int Cl.:
G01N 25/20 (2006.01)         G01N 27/18 (2006.01)
G01N 33/28 (2006.01)

(21) Application number: 09251800.0

(22) Date of filing: 15.07.2009

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA RS

(30) Priority: 16.07.2008 JP 2008184921

(71) Applicant: Mitsui Mining and Smelting Co., Ltd.
Shinagawa-ku,
Tokyo 141-8584 (JP)

(72) Inventors:
• Yanagi, Kiyotaka
Ageo-shi, Saitama 362-0021 (JP)
• Kubota, Akiko
Ageo-shi, Saitama 362-0021 (JP)
• Makino, Tsutomu
Ageo-shi, Saitama 362-0021 (JP)

(74) Representative: Hall, Matthew Benjamin
Frank B. Dehn & Co.
St Bride's House
10 Salisbury Square
London
EC4Y 8JD (GB)

(54) **Fluid identification method and fluid identification apparatus**

(57) A fluid identification sensor is provided that includes a fluid identification element and a fluid temperature detecting element that is disposed separately at a predefined distance from the fluid identification element. A voltage is applied to the fluid identification element for a prescribed time to heat a identification target fluid. A first output value that is an electrical output value corresponding to a first temperature of a fluid identification element and a second output value that is an electrical output value corresponding to a second temperature of a fluid identification element are obtained. A fluid identification is carried out by comparing a rate of change of the first output value and the second output value with a rate of change of a first output value and a second output value for a reference fluid, which has been measured in advance.

[Fig. 1]

**Description**

The present invention relates to a fluid identification method and a fluid identification apparatus for identifying a fluid such as a hydrocarbon liquid such as a gasoline, a naphtha, a kerosene, a light oil, and a heavy oil, and an alcohol liquid such as ethanol and methanol, and a liquid, a gas, and a particulate of an urea aqueous solution. More specifically, the present invention relates to a fluid identification method and a fluid identification apparatus for carrying out a fluid identification such as the fluid type identification, a concentration identification, and the existence or nonexistence identification for a identification target fluid.

While a fuel to be used is assumed, an internal combustion engine of an automobile is designed in such a manner that the automobile is optimally operated in the case in which the fuel is used. For instance, a diesel engine is designed in such a manner that an automobile is optimally operated by using the light oil as a fuel. However, an automobile can be operated even in the case in which a fuel other than the light oil, for instance a wide variety of liquid fuels such as a kerosene and a heavy oil, is used.

Consequently, in the case in which a diesel engine of a construction machine or a heavy machine is operated, a liquid fuel which is comparatively moderate in price as compared with a kerosene, a light oil, and a heavy oil is used without modification in some cases, or the liquid fuel is mixed to a light oil to be used in particular.

However, in the case in which the kerosene having a lubricating property (a viscous property) lower than that of a light oil is mixed to be used, a part of a diesel engine is worn away. Moreover, in the case in which the diesel engine is used over a long period of time, a failure of the diesel engine may occur.

For a construction machine and a heavy machine in particular, a user uses a construction machine or a heavy machine on lease from a trader of a construction machine and a heavy machine as a practical matter. Consequently, after a construction machine or a heavy machine is returned from a user to a lease trader, a failure of an engine may occur in some cases.

In such a case, since a failure of an engine does not occur during use, it is difficult for a lease trader to pursue user's responsibility for the failure in some cases.

Consequently, a request for a sensor that monitors a type of oil in a fuel tank has been increasing.

The present invention was made in order to solve the above problems of the conventional art and to achieve the purpose.

A fluid identification method in accordance with the present invention is for identifying a identification target fluid, and is characterized by comprising the steps of:

> using a fluid identification sensor that includes a fluid identification element;
> applying a voltage for a prescribed time to the fluid identification element to heat a identification target fluid;
> obtaining a first output value that is an electrical output value corresponding to a first temperature of a fluid identification element and a second output value that is an electrical output value corresponding to a second temperature of a fluid identification element; and
> carrying out a fluid identification by comparing a rate of change of the first output value and the second output value with a rate of change of a first output value and a second output value for a reference fluid that has been measured.

The fluid identification method in accordance with the present invention is **characterized in that** the fluid identification sensor further includes a fluid temperature detecting element that is disposed separately at a predefined distance from the fluid identification element.

The fluid identification method in accordance with the present invention is **characterized in that** a fluid identification is carried out based on a difference between the rate of change of a first output value and a second output value for the reference fluid and the rate of change of the first output value and the second output value for the identification target fluid.

The fluid identification method in accordance with the present invention is **characterized in that** an output value of the fluid identification sensor is corrected in such a manner that the rate of change of a first output value and a second output value for the reference fluid is 0.

The fluid identification method in accordance with the present invention is **characterized in that** a fluid identification is carried out based on a ratio of the rate of change of the first output value and the second output value for the identification target fluid to the rate of change of a first output value and a second output value for the reference fluid.

The fluid identification method in accordance with the present invention is **characterized in that** an output value of the fluid identification sensor is corrected in such a manner that the rate of change of a first output value and a second output value for the reference fluid is 1.

The fluid identification method in accordance with the present invention is **characterized in that** the rate of change is an average rate of change.

The fluid identification method in accordance with the present invention is **characterized in that** the first temperature is an initial temperature before a voltage is applied to the fluid identification element.

The fluid identification method in accordance with the present invention is **characterized in that** a difference between the first temperature and the second temperature is at least 20°C.

The fluid identification method in accordance with the present invention is **characterized in that** a difference between the first temperature and the second temperature is at least 40°C.

The fluid identification method in accordance with the present invention is **characterized in that** the fluid identification element is provided with an electrical heating element and a temperature sensing element that is disposed close to the electrical heating element.

The fluid identification method in accordance with the present invention is **characterized in that** the fluid identification element is provided with a temperature sensing element that has a heat generating function and a temperature sensing function.

The fluid identification method in accordance with the present invention is **characterized in that** the fluid identification element and the fluid temperature detecting element are disposed horizontally to a fluid level.

The fluid identification method in accordance with the present invention is **characterized in that** a identification of the identification target fluid is at least one of the fluid type identification, a concentration identification, and the fluid existence or nonexistence identification.

The fluid identification method in accordance with the present invention is **characterized in that** the identification target fluid is a hydrocarbon liquid.

The fluid identification method in accordance with the present invention is **characterized in that** the identification target fluid is at least one of the light oil, the kerosene, and the heavy oil.

A fluid identification apparatus in accordance with the present invention is for identifying a identification target fluid, and is characterized by comprising:

a fluid identification sensor that includes a fluid identification element and a fluid temperature detecting element that is disposed separately at a predefined distance from the fluid identification element; and
a identification control part that discriminates a fluid based on an output from the fluid identification sensor,
wherein a voltage is applied to the fluid identification element for a prescribed time to heat a identification target fluid; a first output value that is an electrical output value corresponding to a first temperature of a fluid identification element and a second output value that is an electrical output value corresponding to a second temperature of a fluid identification element are obtained; and
a fluid identification is carried out by comparing a rate of change of the first output value and the second output value with a rate of change of a first output value and a second output value for a reference fluid, which has been measured and has been stored into the identification control part.

By the present invention, a fluid identification such as a fluid type identification, a concentration identification, and the existence or nonexistence identification for a identification target fluid can be carried out with accuracy by utilizing a first output value of a fluid identification element corresponding to a first temperature of a identification target fluid, a second output value of a fluid identification element corresponding to a second temperature of a identification target fluid, and a rate of change of the first output value and second output value.

Embodiments (examples) of the present invention will be described below in detail with reference to the drawings, in which:

Fig. 1 is an exploded view showing a first embodiment of a fluid identification apparatus in accordance with the present invention;
Fig. 2 is an enlarged view showing a fluid identification sensor module of the fluid identification apparatus of Fig. 1;
Fig. 3 is a schematic cross sectional view showing the fluid identification sensor module of Fig. 2;
Fig. 4 is a schematic cross sectional view showing a usage state of the fluid identification apparatus in accordance with the present embodiment;
Fig. 5 is an exploded schematic view showing a thin film chip of a fluid identification element;
Fig. 6 is a circuit block diagram for a fluid identification;
Fig. 7 is a view showing a relationship between a single pulse voltage P that is applied to an electrical heating element and a sensor output Q;
Fig. 8 is a graph showing a relationship between a temperature and a sensor output value in the case in which an average sensor output value is measured using the fluid identification apparatus in accordance with the present invention for a kerosene, a light oil, an A heavy oil, and a special third light oil;
Fig. 9 is a graph showing a difference in an average rate of change of a first output value V1 and a second output value V2 of each fuel F to be identified;
Fig. 10 is a graph showing a ratio of an average rate of change of a first output value V1 and a second output value V2 of each fuel F to be identified;

Fig. 11 is another circuit block diagram for a fluid identification;

Fig. 12 is a graph showing a difference in an average rate of change on the light oil A basis based on the results of Tables 1 to 4;

Fig. 13 is a graph showing a difference in an average rate of change on the kerosene A basis based on the results of Tables 1 to 4;

Fig. 14 is a graph showing a difference in an average rate of change on a special third light oil basis based on the results of Tables 1 to 4;

Fig. 15 is a graph showing a ratio of an average rate of change on the light oil A basis based on the results of Tables 1 to 4;

Fig. 16 is a graph showing a difference in a simple rate of change on the light oil A basis based on the results of Tables 1 to 4;

Fig. 17 is a graph showing a difference in an average rate of change on the light oil A basis based on the results of Tables 5 to 8;

Fig. 18 is a graph showing a difference in an average rate of change on the kerosene A basis based on the results of Tables 5 to 8;

Fig. 19 is a graph showing a difference in an average rate of change on a special third light oil basis based on the results of Tables 5 to 8;

Fig. 20 is a graph showing a ratio of an average rate of change on the light oil A basis based on the results of Tables 5 to 8;

Fig. 21 is a graph showing a difference in a simple rate of change on the light oil A basis based on the results of Tables 5 to 8;

Fig. 22 is a schematic view showing a second embodiment of a fluid identification apparatus in accordance with the present invention;

Fig. 23 is a schematic view showing an example of a usage state of the fluid identification apparatus of Fig. 22; and

Fig. 24 is a schematic view showing an example of another usage state of the fluid identification apparatus of Fig. 22.

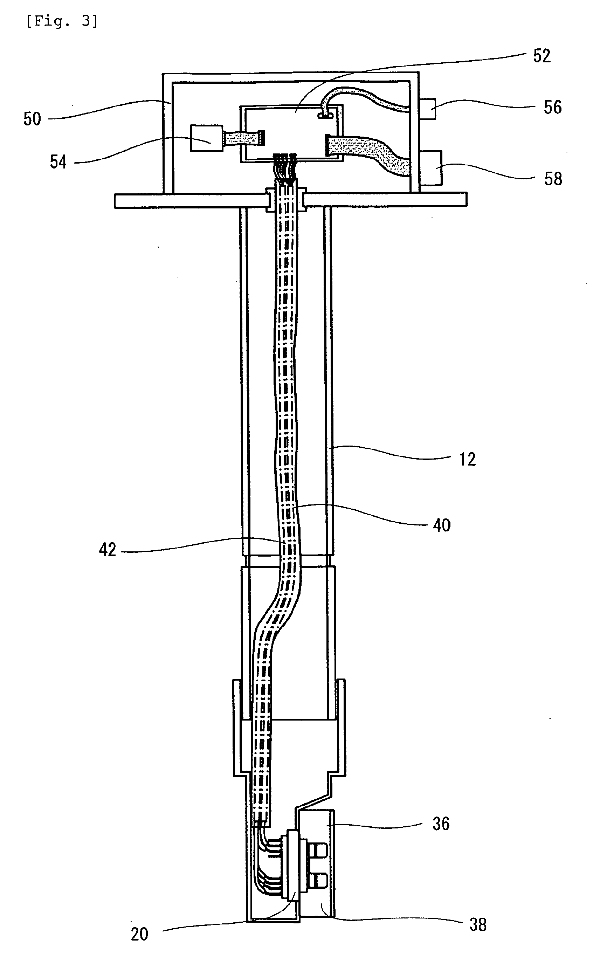

As shown in Fig. 3, a fluid identification apparatus 10 in accordance with an embodiment of the present invention is provided with a support part 12 and is attached to a fuel tank 100 that is mounted to an automobile and a construction machine such as a transporting machine, a bulldozer, and a crane for instance.

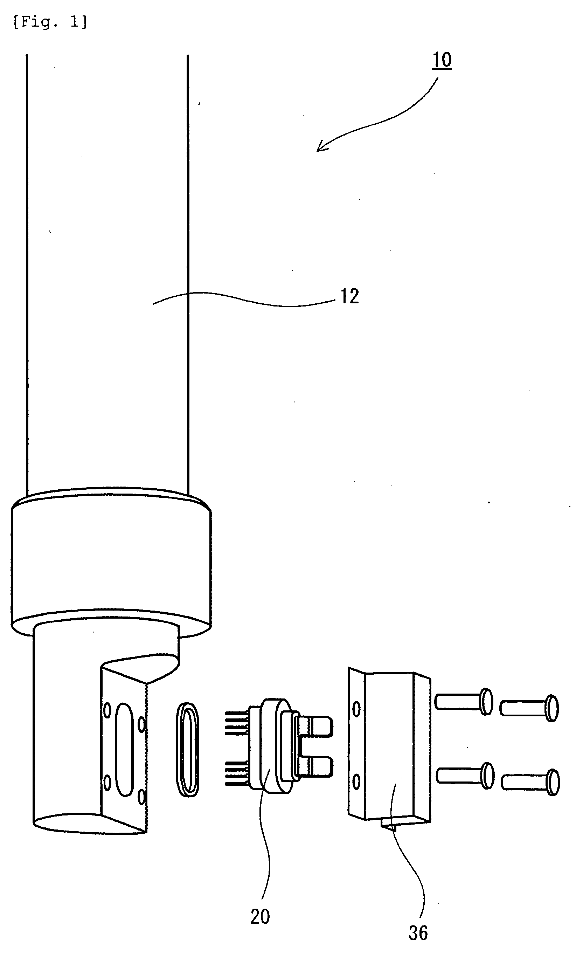

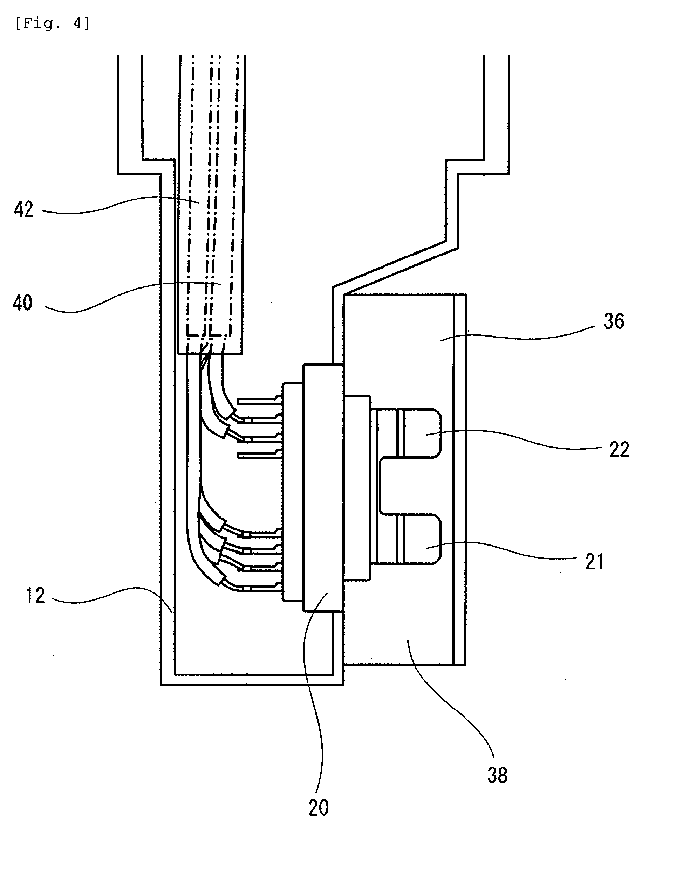

Moreover, as shown in Figs. 1 and 4, the fluid identification apparatus 10 in accordance with an embodiment of the present invention is provided with a fluid identification sensor module 20, a cover member 36, a power cable 40, and a communication cable 42. The fluid identification sensor module 20 is disposed in such a manner that only a side on which a fluid identification element 21 and a fluid temperature detecting element 22 are disposed (hereafter referred to a fluid exposed side) comes into contact with a fluid.

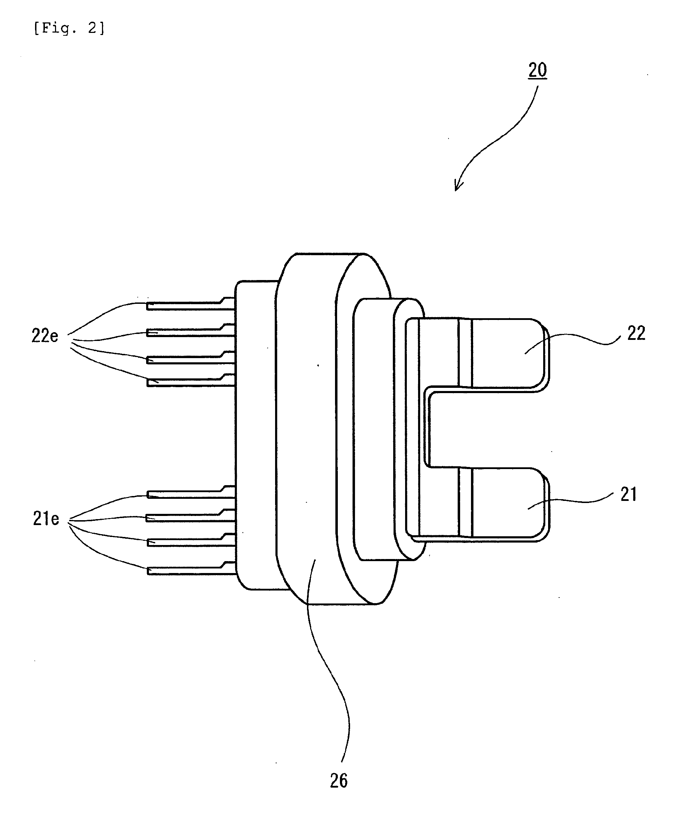

As shown in Fig. 2, the fluid identification sensor module 20 is molded by a mold resin 26 in an integrated manner in a state in which the fluid identification element 21 and the fluid temperature detecting element 22 are disposed separately at a predefined distance from each other. A numeral 21e represents an external electrode terminal that is electrically connected to the fluid identification element 21, and a numeral 22e represents an external electrode terminal that is electrically connected to the fluid temperature detecting element 22.

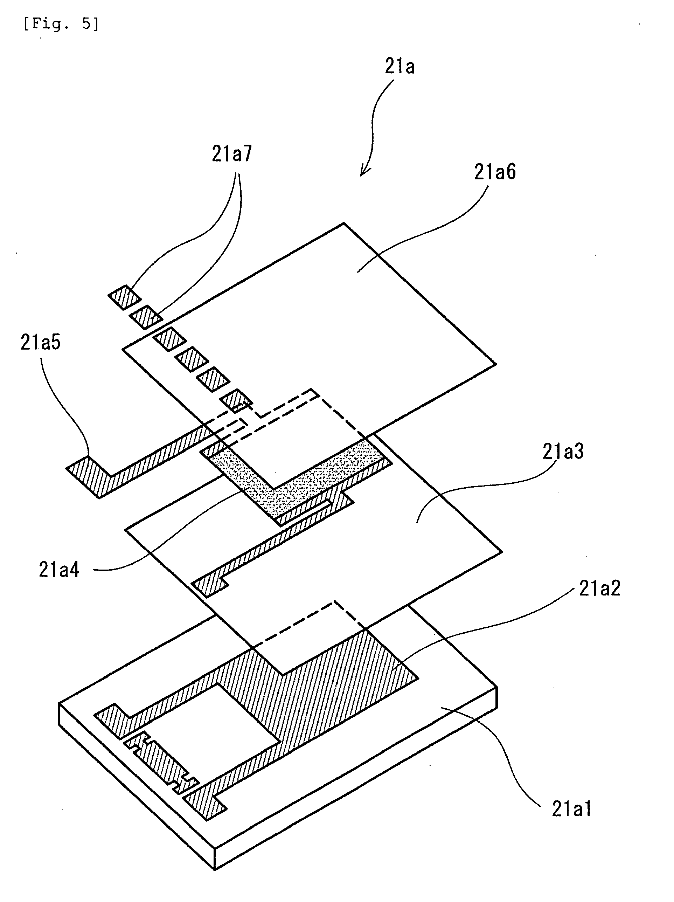

In the embodiment of the present invention, as shown in Fig. 5, the fluid identification element 21 is configured by a fluid detecting thin film chip 21a in which a fluid detecting temperature sensing element is formed by a thin film on a chip substrate.

The fluid detecting thin film chip 21a is composed of a chip substrate 21a1 made of $Al_2O_3$, a fluid detecting temperature sensing element 21a2 made of Pt, an interlayer insulation film 21a3 made of $SiO_2$, an electrical heating element 21a4 made of $TaSiO_2$, an electrical heating element electrode 21a5 made of Ni, a protective film 21a6 made of $SiO_2$, and an electrode pad 21a7 made of Ti/Au, which are laminated in the order as needed for instance. Although this is not shown in the figure, the fluid detecting temperature sensing element 21a2 is formed in a meandering pattern.

The electrode pad 21a7 that is connected to the fluid detecting temperature sensing element 21a2 and the electrical heating element electrode 21a5 is connected to the external electrode terminal 21e via a bonding wire 21d.

Moreover, the fluid temperature detecting element 22 can also be configured similarly to the fluid identification element 21. However, only a temperature sensing element (a fluid temperature detecting temperature sensing element for the fluid temperature detecting element 22) is operated without operating an electrical heating element. A fluid temperature detecting element 22 in which an electrical heating element and an electrical heating element electrode are not formed can also be used unlike a fluid temperature detecting element for a fluid identification element.

Moreover, as shown in Figs. 3 and 4, the external electrode terminal 21e of the fluid identification element 21 and the external electrode terminal 22e of the fluid temperature detecting element 22 are connected to a power cable 40 and a communication cable 42, respectively.

The power cable 40 and the communication cable 42 are extended upward through the inside of the support part 12, and are connected to a control unit 50 that is disposed outside the fuel tank 100 and that configures a identification control part.

The control unit 50 is provided with an ASIC (Application Specific Integrated Circuit) 52 that carries out a control of voltage application to the fluid identification sensor module 20 and a identification of a fluid based on an electrical output of the fluid identification sensor module 20, a storage device 54 for stores the fluid identification data that has been measured in advance, a power connection terminal 56 for a power input, and a CAN interface 58 for carrying out a CAN (Cable Area Network) communication.

Moreover, as shown in Figs. 1 and 4, a cover member 36 is attached to the fluid identification apparatus 10 in such a manner that the fluid identification sensor module 20 is surrounded by the cover member 36. A identified fluid introduction path 38 that has the both upper and lower ends opened and that is extended in a vertical direction through a region close to a fluid exposed side of the fluid identification sensor module 20 is formed by the cover member 36.

In the embodiment of the present invention, the fluid identification element 21 and the fluid temperature detecting element 22 of the fluid identification sensor module 20 are disposed vertically to a fluid level, and the identified fluid introduction path 38 has the both upper and lower ends that are opened. However, the fluid identification element 21 and the fluid temperature detecting element 22 of the fluid identification sensor module 20 can also be disposed horizontally to a fluid level, and the identified fluid introduction path 38 can also have the both right and left ends that are opened.

By horizontally disposing the fluid identification element 21 and the fluid temperature detecting element 22 as described above, a difference between a temperature distribution of a identification target fluid around the fluid identification element 21 and a temperature distribution of a identification target fluid around the fluid temperature detecting element 22 can be reduced.

The fluid identification apparatus in accordance with the present invention carries out a identification of a fluid based on a temperature change of a identification target fluid as described later. Consequently, by disposing the fluid identification element 21 and the fluid temperature detecting element 22 horizontally to a fluid level, a difference in a temperature distribution can be reduced, thereby improving the identification accuracy.

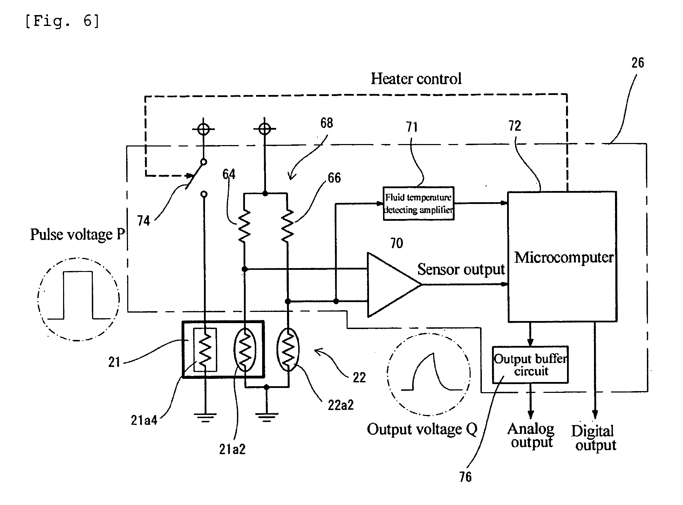

Fig. 6 is a circuit block diagram for showing a circuit configuration of an ASIC 52 for a fluid identification in accordance with the embodiment of the present invention. A bridge circuit (a fluid identification circuit) 68 is configured by the temperature sensing element 21a2 of the fluid identification element 21, the temperature sensing element 22a2 of the fluid temperature detecting element 22, and two resistors 64 and 66. An output of the bridge circuit 68 is input to a differential amplifier 70, and an output of the differential amplifier 70 (also called a fluid identification circuit output or a sensor output) is input to a microcomputer 72 that configures a computing part via an A/D converter.

A fluid temperature corresponding output value that is corresponded to a temperature of a identification target fluid is input from the temperature sensing element 22a2 of the fluid temperature detecting element 22 to the microcomputer 72 via a fluid temperature detecting amplifier 71. On the other hand, the microcomputer 72 outputs a heater control signal that controls the opening and closing of a switch 74 to the switch 74 that is located a power distribution path to the electrical heating element 21a4 of the fluid identification element 21.

A part that is surrounded by an alternate long and short dash line in Fig. 6 is formed in the ASIC 52.

Fig. 6 shows a configuration in which the switch 74 is simply opened and closed as a matter of practical convenience. However, a plurality of voltage application paths that can apply voltages different from each other can also be formed in a fabrication of the ASIC 52, and any of the voltage application paths can be selected in the case in which a heater is controlled.

By the above configuration, a range of selecting a characteristic of the electrical heating element 21a4 of the fluid identification element 21 can be extremely enlarged. In other words, a voltage that is optimum for a measurement can be applied corresponding to the characteristics of the electrical heating element 21a4. Moreover, a plurality of voltage applications different from each other can be carried out in the case in which a heater is controlled, whereby a range of types of a identification target fluid can be enlarged.

Fig. 6 shows the resistors 64 and 66 having a fixed resistance value as a matter of practical convenience. However, variable resistors can be formed as the resistors 64 and 66 in the case in which the ASIC 52 is formed, and the resistance values of the resistors 64 and 66 can be changed as needed in a measurement.

Similarly, the differential amplifier 70 and the fluid temperature detecting amplifier 71 can be formed in such a manner that the characteristics of the differential amplifier 70 and the fluid temperature detecting amplifier 71 can be adjusted in the case in which the ASIC 52 is formed, and the characteristics of the amplifiers can be changed as needed in a measurement.

By the above configuration, the characteristics of the fluid identification circuit can be easily set to be optimum, and a dispersion of the measurement characteristics, which occurs based on an individual dispersion on a production of the fluid identification element 21 and the fluid temperature detecting element 22 and an individual dispersion on a production of the ASIC 52, can be reduced, thereby improving a production yield.

As an example of the fluid identification apparatus in accordance with the embodiment of the present invention, an oil type identification operation of a light oil, a kerosene, and a heavy oil will be described in the following.

In the case in which a fuel F to be identified is stored into the fuel tank 100, the fuel F to be identified is also filled with in the identified fluid introduction path 38 that is formed by the cover member 36 that covers the fluid identification sensor module 20. The fuel F to be identified that has been stored into the fuel tank 100 and the identified fluid introduction path 38 does not flow in substance.

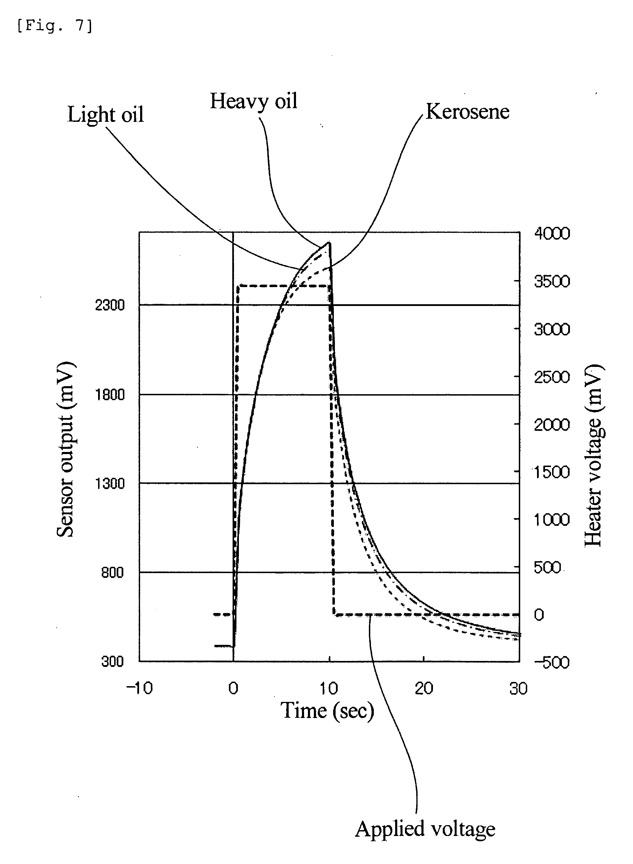

The switch 74 is closed for a prescribed time (10 seconds for instance) by a heater control signal that is output from the microcomputer 72 to the switch 74, and a single pulse voltage P of a prescribed height (3.45 V for instance) is applied to the electrical heating element 21a4 to make the electrical heating element generate a heat. As shown in Fig. 7, an output voltage (a sensor output) of the differential amplifier 70 at this time is increased by a gradual process in a voltage application to the electrical heating element 21a4, and is decreased by a gradual process after a voltage application to the electrical heating element 21a4 is completed.

As shown in Fig. 7, immediately before a voltage application to the electrical heating element 21a4 is completed, the microcomputer 72 carries out a sampling of prescribed numbers (256 times for instance) and carries out an operation for getting the average value to obtain an average sensor output voltage value. The average sensor output voltage value is corresponded to a peak temperature of the temperature sensing element 21a2.

It is not always necessary that an average sensor output voltage value is a value that is corresponded to a peak temperature of the temperature sensing element 21a2. The microcomputer 72 can also sample a sensor output after a prescribed time (5 seconds for instance) elapses from a start of a voltage application to the electrical heating element 21a4, and can carry out an operation for getting the average value to obtain an average sensor output voltage value.

For the meanwhile, a heat that has been generated by the electrical heating element 21a4 based on a voltage application of a single pulse as described above is transferred to a identification target fluid. By this heat transfer, a identification target fluid around the fluid identification sensor module 20 is heated, and a temperature of the identification target fluid is increased. The heat transfer depends on a specific gravity, a coefficient of kinematic viscosity, and a lubricity (HFRR: High Frequency Reciprocating Rig) of the identification target fluid. A degree of a temperature increase of the temperature sensing element 21a2 is changed by a type and a concentration of a fluid and a temperature of a fluid.

The average sensor output voltage value that can be obtained as described above is measured for a first average sensor output voltage value (a first output value V1) that is corresponded to a first temperature T1 of the fuel F to be identified and for a second average sensor output voltage value (a second output value V2) that is corresponded to a second temperature T2.

Here, the larger a difference in a temperature between the first temperature T1 and the second temperature T2 is, the higher an accuracy of the measurement is. It is preferable that the difference in a temperature is at least 20°C, more preferably at least 40°C.

The fuel F to be identified can be heated from the first temperature T1 to the second temperature T2 by using the electrical heating element 21a4 of the fluid identification element 21. The fuel F to be identified can also be heated by a heater that is prepared separately. Moreover, the fuel tank 100 can be disposed in a constant temperature reservoir such as an incubator. The fuel tank 100 can also be a constant temperature water tank.

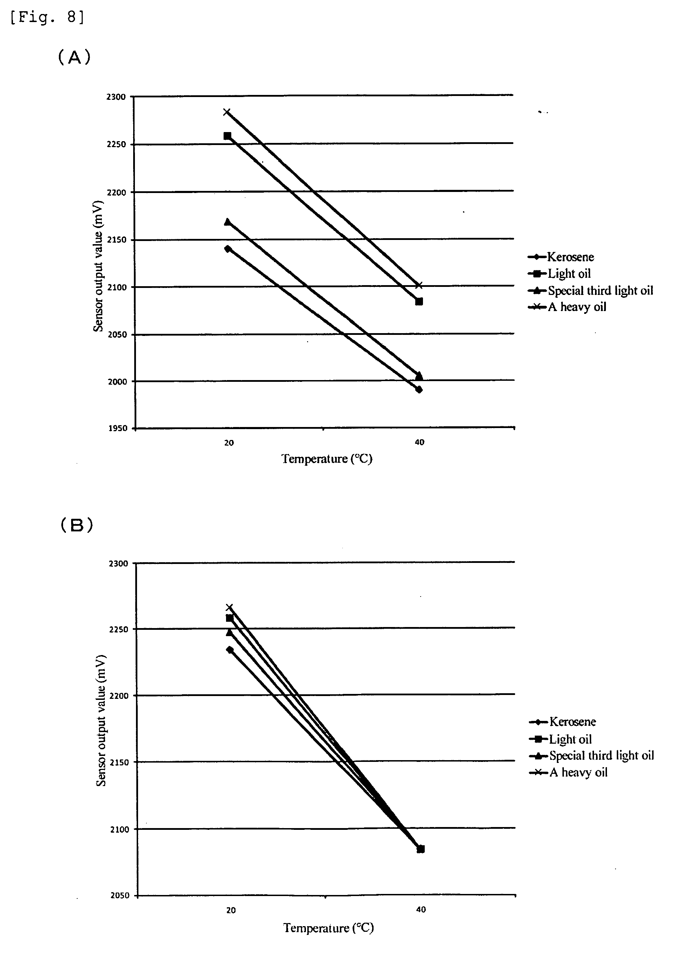

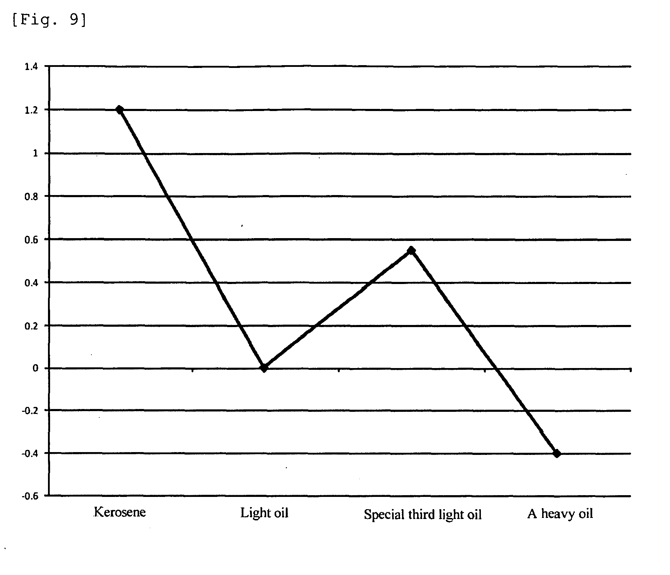

Fig. 8 is a graph showing a relationship between a temperature and a sensor output value in the case in which an average sensor output value is measured using the fluid identification apparatus in accordance with the present invention for a kerosene, a light oil, an A heavy oil, and a special third light oil. Fig. 9 is a graph showing a difference in an average rate of change of a first output value V1 and a second output value V2 of each fuel F to be identified.

In Fig. 8, the first temperature T1 is set to 0°C and the second temperature T2 is set to 20°C. Fig. 8(A) is a graph in which the obtained output values are plotted without modification. Fig. 8(B) is a graph in which the values of the second output value V2 of the fuel F to be identified are corrected to a constant value, that is the second output value V2 of a light oil in Fig. 8(B), and are plotted.

As shown in Fig. 8, a rate of change of the sensor output corresponding to a temperature change, that is a slope of a graph, is different depending on a type of the fuel F to be identified.

A type of the fuel F to be identified can be identified by the rate of change of the sensor output.

Fig. 9 is a graph showing a difference in a rate of change of a sensor output of each fuel F to be identified, that is a difference in a rate of change of each fuel F to be identified on a light oil basis, in the case in which a rate of change of the sensor output for a light oil is corrected to 0.

As described above, by using the light oil as a basis for instance, a rate of change of each fuel F to be identified becomes a specific value depending on the type thereof. Consequently, a type of the fuel F to be identified can be identified by measuring the rate of change of the sensor output of the fuel F to be identified.

A fluid that is used as a basis is not restricted to the light oil, and any fluid can be used as a basis, not to be argued.

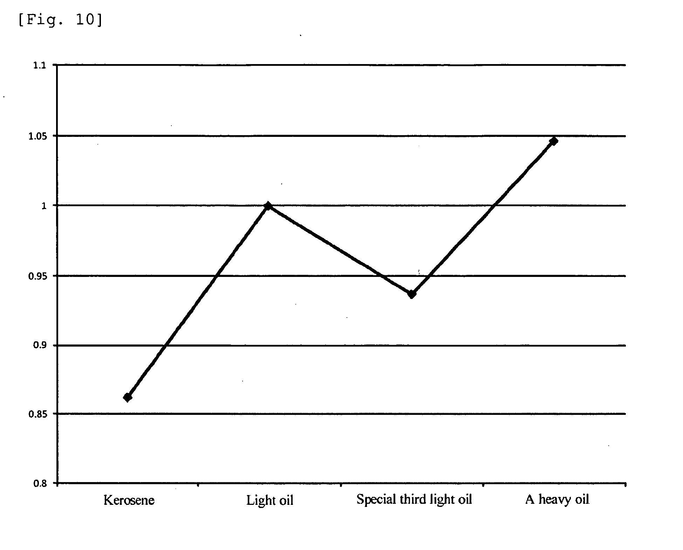

Fig. 10 is a graph showing a ratio of a rate of change of a sensor output of each fuel F to be identified, that is a ratio of a rate of change of each fuel F to be identified on a light oil basis, in the case in which a rate of change of the sensor output for a light oil is corrected to 1.

As described above, even in the case in which a ratio of a rate of change is used, the characteristics for each fuel to be identified can be found similarly to the case of Fig. 9. Consequently, a type of the fuel F to be identified can be identified by measuring the rate of change of the sensor output of the fuel F to be identified.

A fluid that is used as a basis is not restricted to the light oil, and any fluid can be used as a basis, not to be argued.

In the present embodiment, an average rate of change is used as a rate of change as shown in the numerical expression 1. However, a simple rate of change as shown in the numerical expression 2 can also be used for carrying out the identification.

[Numerical expression 1]

$$\text{Average rate of change} = (V2-V1) / (T2-T1)$$

[Numerical expression 2]

$$\text{Simple rate of change} = (V2-V1) / V1$$

Only the identification of a type of a fuel is described in the present embodiment. However, a concentration of a fuel and the existence or nonexistence of a fuel can also be identified by obtaining an output value and by obtaining a difference in a rate of change and a ratio of a rate of change similarly.

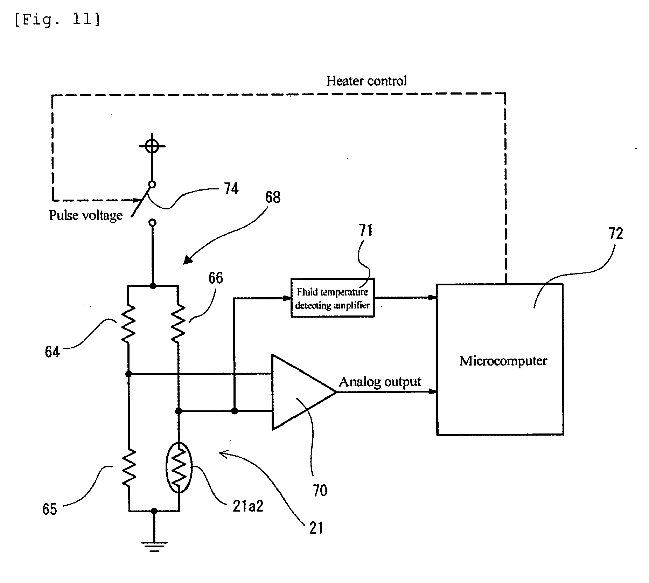

Fig. 11 is another circuit block diagram of an ASIC 52 for a fluid identification for a fluid identification apparatus in accordance with the present invention.

In the ASIC 52 shown in Fig. 11, a power distribution to a bridge circuit (a fluid identification circuit) 68 that is configured by the temperature sensing element 21a2 and three resistors 64, 65 and 66 of the fluid identification element 21 by controlling the opening and closing of a switch 74 by the power distribution control signal that is output from the microcomputer 72.

In the present embodiment, the temperature sensing element 21a2 of the fluid identification element 21 is made of a substance in which an electrical resistance value is changed due to a temperature, such as Pt, Ni, Cr, and W as a metal material, NiCr, FeCr as an alloy material, NiO, FeO, CuO, and $Ni_2O_3$ as an oxide material, and $TaSiO_2$ and $CrSiO_2$ as a cermet.

By the above configuration, by the self heating of the temperature sensing element 21a2, a resistance value of the temperature sensing element 21a2 is changed, a balance of the bridge circuit 68 is broken up, and an output voltage (sensor output) Q is output via the differential amplifier 70 similarly to Fig. 7.

Consequently, similarly to the ASIC shown in Fig. 6, an average sensor output voltage value can be obtained and a fluid identification of a identification target fluid can be carried out as described above.

In the case in which a fluid identification is carried out by the ASIC 55 and the temperatures T1 and T2 of a identification target fluid are measured, an electrical output is obtained via a fluid temperature detecting amplifier 71 based on the resistance value of the temperature sensing element 21a2 of the fluid identification element 21.

For the ASIC 55, an output voltage Q is obtained by creating a difference in a temperature change of a resistance value of the temperature sensing element 21a2 and the resistor 65 by using a material different from the resistor 65 as a material of the temperature sensing element 21a2. However, the present invention is not restricted to this configuration. For instance, the output voltage Q can also be obtained by setting a resistance value of the temperature sensing element 21a2 to be larger than a resistance value of the resistor 65 and by setting a calorific value of the temperature sensing element 21a2 to be larger than a calorific value of the resistor 65 to break up a balance of the bridge circuit 68 in accordance with a power distribution to the bridge circuit 68. Moreover, the resistor 65 can also be dipped into the same liquid together with the fluid identification element 21 as a fluid temperature detecting element 22 in order to improve the accuracy.

A sensor output voltage value and a rate of change in the case in which a plurality of fluids to be identified by using the fluid identification apparatus 10 in accordance with the present invention are identified as a practical matter are shown in the following.

Tables 1 to 4 lists the measured values for the kerosene A, the kerosene B, the light oil A, the light oil B, the special third light oil, the A heavy oil A, and the A heavy oil B in the case in which the first temperature T1 is 20°C and the second temperature T2 is 40°C. In the present embodiment, four sensors (O-16 to O-19) that have the same configuration are used and the results thereof are shown.

[Table 1]

| Oil type | Temperature (°C) | Concentration (%) | Sensor output voltage value (mV) |
|---|---|---|---|
| Kerosene A | 22. 1 | 9. 40 | 2140. 0 |
| | 41. 4 | 14. 00 | 1989. 9 |
| Kerosene B | 22. 1 | 8. 21 | 2133. 9 |
| | 41. 3 | 13. 10 | 1984. 4 |
| Light oil A | 22. 2 | 33. 29 | 2258. 1 |
| | 41. 5 | 33. 00 | 2083. 8 |
| Light oil B | 22. 0 | 31. 00 | 2247. 7 |
| | 41. 6 | 31. 10 | 2073. 5 |
| Special third light oil | 21. 9 | 14. 90 | 2168. 4 |
| | 41. 6 | 17. 40 | 2005. 4 |
| A heavy oil A | 22. 2 | 34. 50 | 2264. 0 |
| | 41. 6 | 33. 51 | 2085. 8 |
| A heavy oil B | 22. 2 | 34. 90 | 2266. 1 |
| | 41. 6 | 33. 99 | 2087. 9 |

[Table 2]

| Oil type | Temperature (°C) | Concentration (%) | Sensor output voltage value (mV) |
|---|---|---|---|
| Kerosene A | 22. 0 | 10. 40 | 2159. 0 |
| | 41. 4 | 14. 00 | 2005. 9 |
| Kerosene B | 22. 3 | 9. 20 | 2152. 5 |
| | 41. 5 | 13. 00 | 2000. 1 |
| Light oil A | 22. 4 | 34. 60 | 2277. 1 |
| | 41. 5 | 33. 20 | 2099. 9 |
| Light oil B | 22. 3 | 32. 01 | 2266. 8 |
| | 41. 6 | 31. 20 | 2089. 2 |
| Special third light oil | 22. 2 | 16. 30 | 2189. 2 |
| | 41. 6 | 17. 40 | 2021. 6 |
| A heavy oil A | 22. 4 | 35. 70 | 2283. 0 |
| | 41. 6 | 33. 60 | 2101. 2 |
| A heavy oil B | 22. 4 | 36. 20 | 2284. 9 |
| | 41. 5 | 34. 00 | 2103. 6 |

[Table 3]

| Oil type | Temperature (°C) | Concentration (%) | Sensor output voltage value (mV) |
|---|---|---|---|
| Kerosene A | 22. 0 | | 2105. 5 |
| | 41. 2 | | 1958. 0 |

(continued)

| Oil type | Temperature (°C) | Concentration (%) | Sensor output voltage value (mV) |
|---|---|---|---|
| Kerosene B | 22. 1 | | 2099. 2 |
| | 41. 2 | | 1952. 3 |
| Light oil A | 22. 1 | | 2220. 1 |
| | 41. 2 | | 2049. 3 |
| Light oil B | 22. 0 | | 2210. 1 |
| | 41. 3 | | 2039. 0 |
| Special third light oil | 22. 0 | | 2135. 0 |
| | 41. 3 | | 1973. 2 |
| A heavy oil A | 22. 1 | | 2224. 9 |
| | 41. 3 | | 2050. 5 |
| A heavy oil B | 22. 1 | | 2227. 0 |
| | 41. 3 | | 2052. 4 |

[Table 4]

| Oil type | Temperature (°C) | Concentration (%) | Sensor output voltage value (mV) |
|---|---|---|---|
| Kerosene A | 22. 3 | 9. 60 | 2125. 3 |
| | 41. 4 | 14. 00 | 1976. 3 |
| Kerosene B | 22. 3 | 8. 40 | 2119. 1 |
| | 41. 5 | 13. 00 | 1970. 9 |
| Light oil A | 22. 4 | 33. 00 | 2241. 2 |
| | 41. 5 | 32. 30 | 2068. 7 |
| Light oil B | 22. 1 | 32. 50 | 2231. 9 |
| | 41. 5 | 31. 40 | 2058. 4 |
| Special third light oil | 22. 3 | 16. 19 | 2152. 1 |
| | 41. 6 | 17. 40 | 1991. 8 |
| A heavy oil A | 22. 3 | 36. 10 | 2246. 8 |
| | 41. 6 | 34. 00 | 2070. 2 |
| A heavy oil B | 22. 2 | 36. 50 | 2248. 9 |
| | 41. 5 | 34. 30 | 2072. 2 |

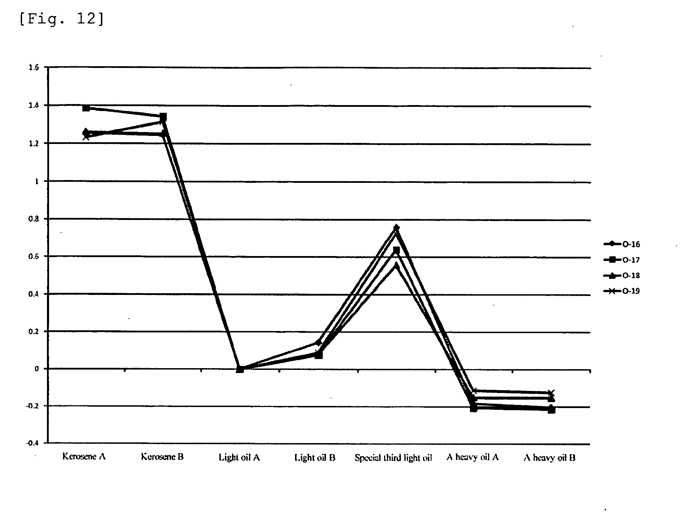

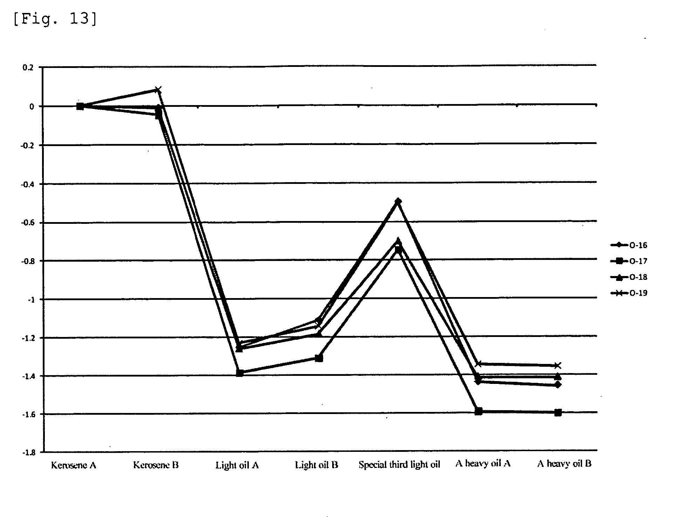

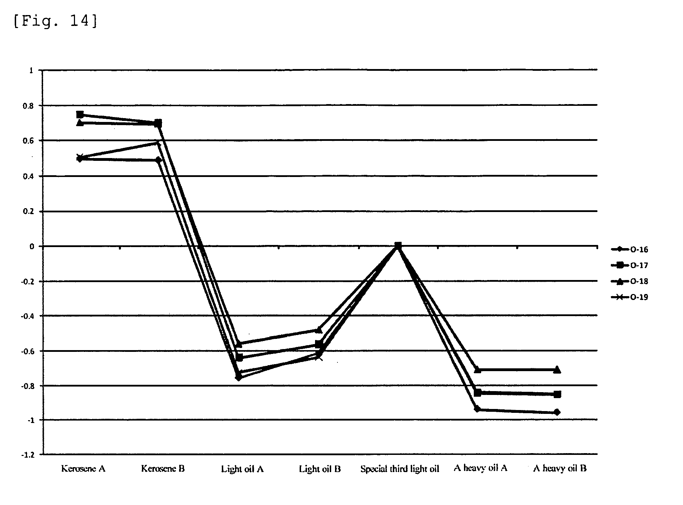

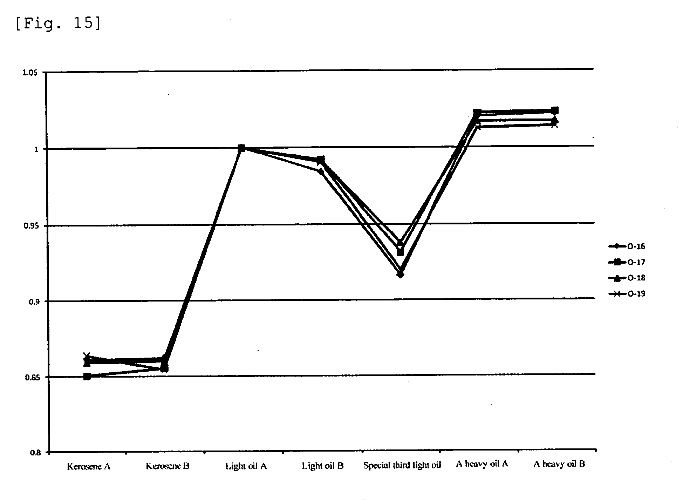

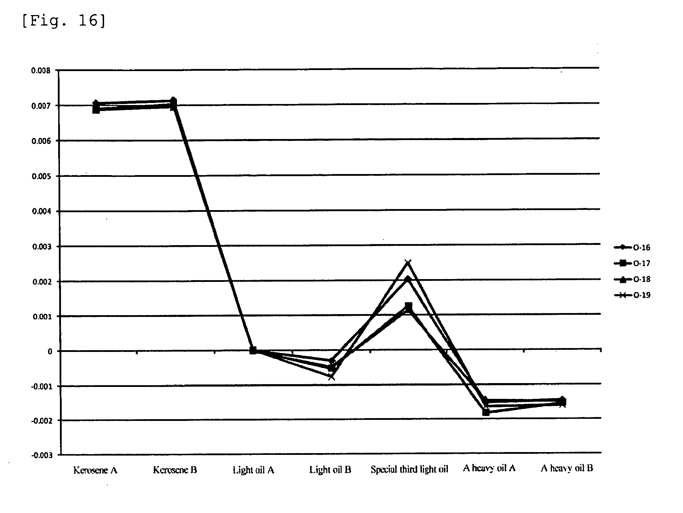

Fig. 12 is a graph showing a difference in an average rate of change on the light oil A basis based on the results of Tables 1 to 4. Fig. 13 is a graph showing a difference in an average rate of change on the kerosene A basis based on the results of Tables 1 to 4. Fig. 14 is a graph showing a difference in an average rate of change on a special third light oil basis based on the results of Tables 1 to 4. Fig. 15 is a graph showing a ratio of an average rate of change on the light oil A basis based on the results of Tables 1 to 4. Fig. 16 is a graph showing a difference in a simple rate of change on the light oil A basis based on the results of Tables 1 to 4.

In Figs. 12 to 16, the characteristics that are apparent by a type of a identification target fluid, that is a difference in a value of a identification target fluid and a value of a fluid to be a basis, can be found. By using the results, a type of a identification target fluid can be identified.

Tables 5 to 8 lists the measured values for the kerosene A, the kerosene B, the light oil A, the light oil B, the special third light oil, the A heavy oil A, and the A heavy oil B in the case in which the first temperature T1 is 0°C and the second temperature T2 is 40°C. In the present embodiment, four sensors (O-16 to O-19) that have the same configuration are also used and the results thereof are shown.

[Table 5]

| Oil type | Temperature (°C) | Concentration (%) | Sensor output voltage value (mV) |
|---|---|---|---|
| Kerosene A | 2. 4 | 3. 20 | 2317. 0 |
| | 41. 4 | 14. 00 | 1989. 9 |
| Kerosene B | 2. 6 | 1. 81 | 2310. 1 |
| | 41. 3 | 13. 10 | 1984. 4 |
| Light oil A | 2. 7 | 32. 81 | 2465. 9 |
| | 41. 5 | 33. 00 | 2083. 8 |
| Light oil B | 2. 5 | 30. 25 | 2454. 1 |
| | 41. 6 | 31. 10 | 2073. 5 |
| Special third light oil | 2. 6 | 9. 19 | 2348. 6 |
| | 41. 6 | 17. 40 | 2005. 4 |
| A heavy oil A | 2. 5 | 36. 40 | 2482. 9 |
| | 41. 6 | 33. 51 | 2085. 8 |
| A heavy oil B | 2. 7 | 36. 90 | 2485. 3 |
| | 41. 5 | 33. 99 | 2087. 9 |

[Table 6]

| Oil type | Temperature (°C) | Concentration (%) | Sensor output voltage value (mV) |
|---|---|---|---|
| Kerosene A | 2. 5 | 1. 80 | 2339. 0 |
| | 41. 4 | 14. 00 | 2005. 9 |
| Kerosene B | 2. 7 | 0. 40 | 2332. 3 |
| | 41. 5 | 13. 00 | 2000. 1 |
| Light oil A | 2. 7 | 31. 90 | 2489. 4 |
| | 41. 5 | 33. 20 | 2099. 9 |
| Light oil B | 2. 7 | 29. 20 | 2476. 4 |
| | 41. 6 | 31. 20 | 2089. 2 |
| Special third light oil | 2. 6 | 7. 90 | 2371. 2 |
| | 41. 6 | 17. 40 | 2021. 6 |
| A heavy oil A | 2. 7 | 35. 20 | 2505. 6 |
| | 41. 6 | 33. 60 | 2101. 2 |
| A heavy oil B | 2. 7 | 36. 00 | 2508. 8 |
| | 41. 5 | 34. 00 | 2103. 6 |

[Table 7]

| Oil type | Temperature (°C) | Concentration (%) | Sensor output voltage value (mV) |
|---|---|---|---|
| Kerosene A | 2. 5 | 2. 20 | 2278. 9 |
| | 41. 4 | 14. 00 | 1958. 0 |
| Kerosene B | 2. 7 | 0. 60 | 2272. 0 |
| | 41. 5 | 12. 90 | 1952. 3 |
| Light oil A | 2. 8 | 32. 20 | 2423. 8 |
| | 41. 5 | 32. 30 | 2049. 3 |
| Light oil B | 2. 7 | 29. 72 | 2412. 0 |
| | 41. 6 | 30. 50 | 2039. 0 |
| Special third light oil | 2. 7 | 9. 00 | 2310. 1 |
| | 41. 6 | 17. 40 | 1973. 2 |
| A heavy oil A | 2. 8 | 34. 91 | 2438. 7 |
| | 41. 6 | 32. 70 | 2050. 5 |
| A heavy oil B | 2. 8 | 35. 50 | 2441. 4 |
| | 41. 5 | 33. 00 | 2052. 4 |

[Table 8]

| Oil type | Temperature (°C) | Concentration (%) | Sensor output voltage value (mV) |
|---|---|---|---|
| Kerosene A | 2. 5 | 2. 20 | 2300. 7 |
| | 41. 4 | 14. 00 | 1976. 3 |
| Kerosene B | 2. 7 | 0. 60 | 2294. 1 |
| | 41. 5 | 13. 00 | 1970. 9 |
| Light oil A | 2. 8 | 32. 20 | 2447. 1 |
| | 41. 5 | 32. 30 | 2068. 7 |
| Light oil B | 2. 7 | 29. 72 | 2435. 5 |
| | 41. 5 | 31. 40 | 2058. 3 |
| Special third light oil | 2. 7 | 9. 00 | 2332. 3 |
| | 41. 6 | 17. 40 | 1991. 8 |
| A heavy oil A | 2. 7 | 35. 81 | 2463. 2 |
| | 41. 6 | 34. 00 | 2070. 2 |
| A heavy oil B | 2. 7 | 36. 60 | 2466. 7 |
| | 41. 5 | 34. 30 | 2072. 2 |

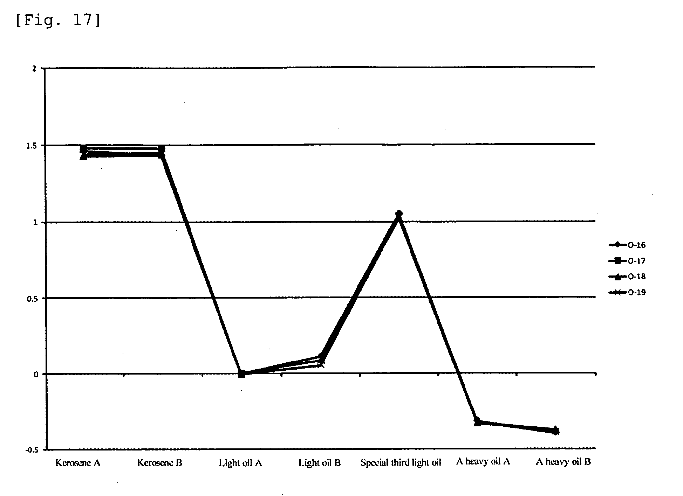

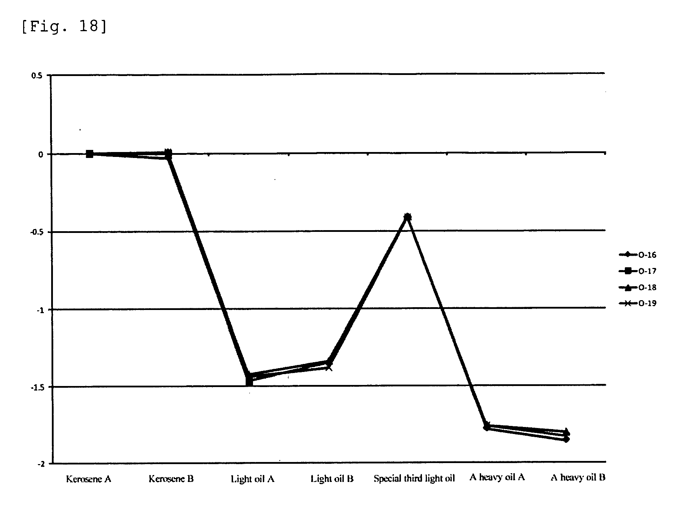

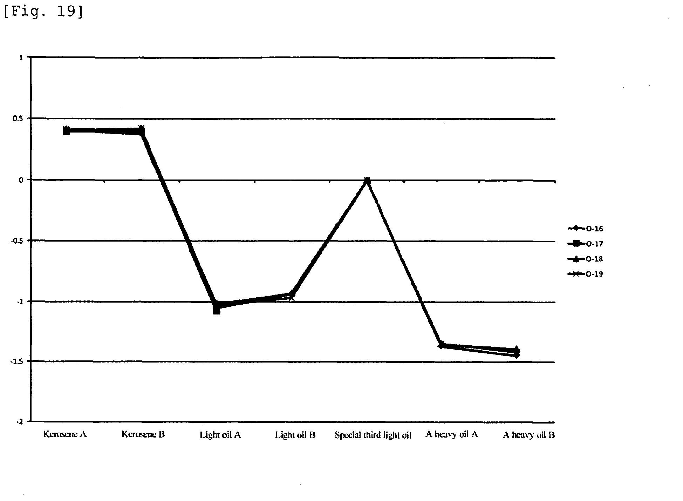

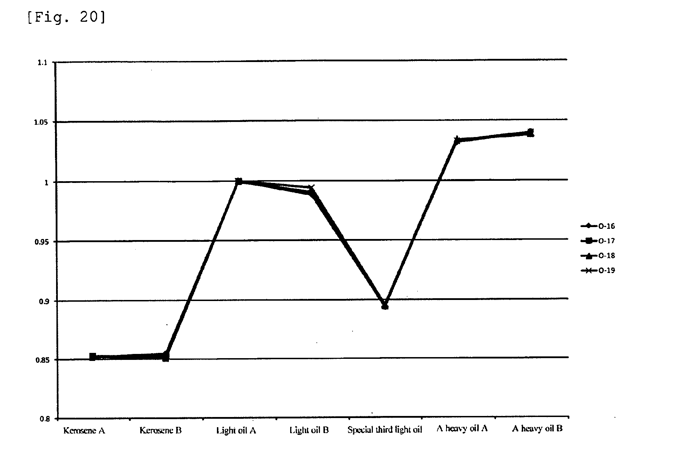

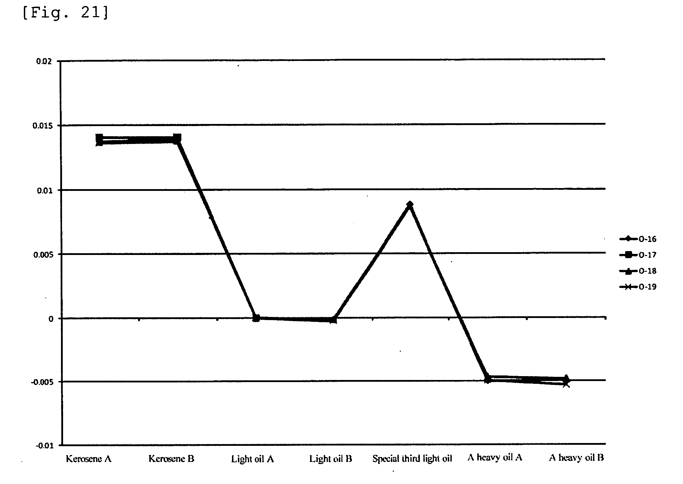

Fig. 17 is a graph showing a difference in an average rate of change on the light oil A basis based on the results of Tables 5 to 8. Fig. 18 is a graph showing a difference in an average rate of change on the kerosene A basis based on the results of Tables 5 to 8. Fig. 19 is a graph showing a difference in an average rate of change on a special third light oil basis based on the results of Tables 5 to 8. Fig. 20 is a graph showing a ratio of an average rate of change on the light oil A basis based on the results of Tables 5 to 8. Fig. 21 is a graph showing a difference in a simple rate of change on the light oil A basis based on the results of Tables 5 to 8.

As compared with the cases of Figs. 12 to 16 in which a difference in a temperature of the first temperature T1 and the second temperature T2 is 20°C, a difference in the results for each sensor is not found in Figs. 17 to 21. This means that an error of measurement for each sensor is reduced and the accuracy of a sensor output value is improved. In other words, by increasing a difference in a temperature of the first temperature T1 and the second temperature T2, the identification accuracy of a fluid identification can be improved.

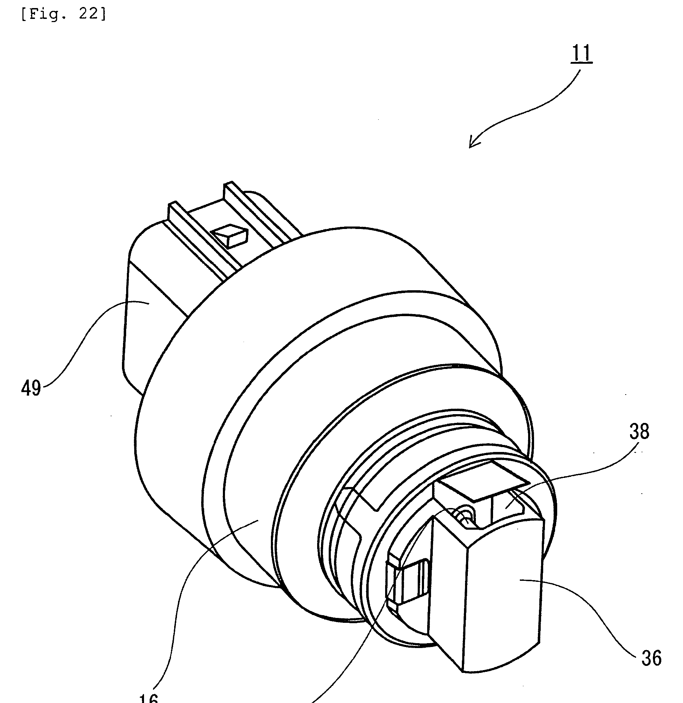

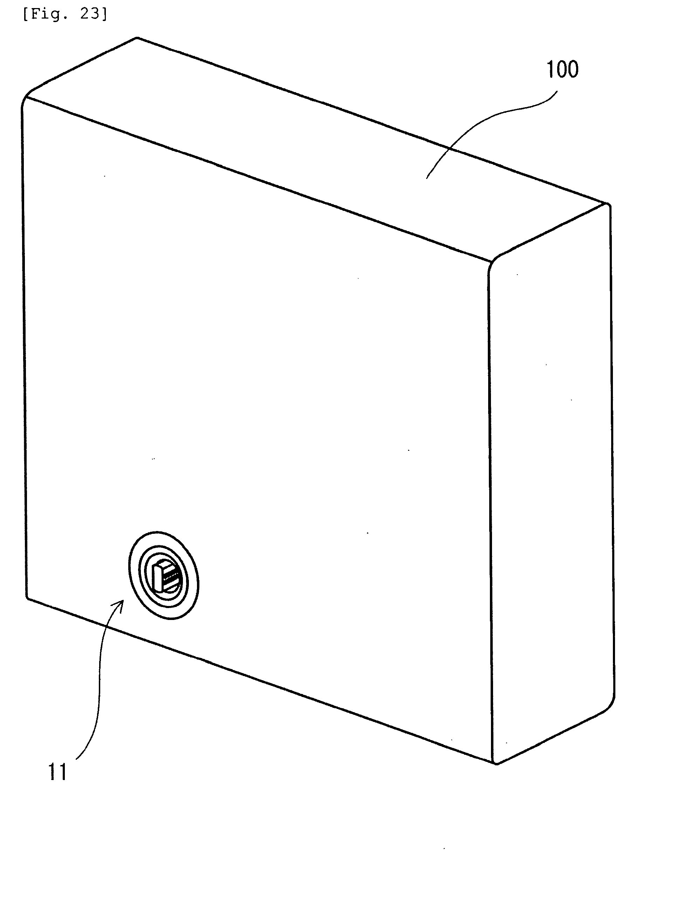

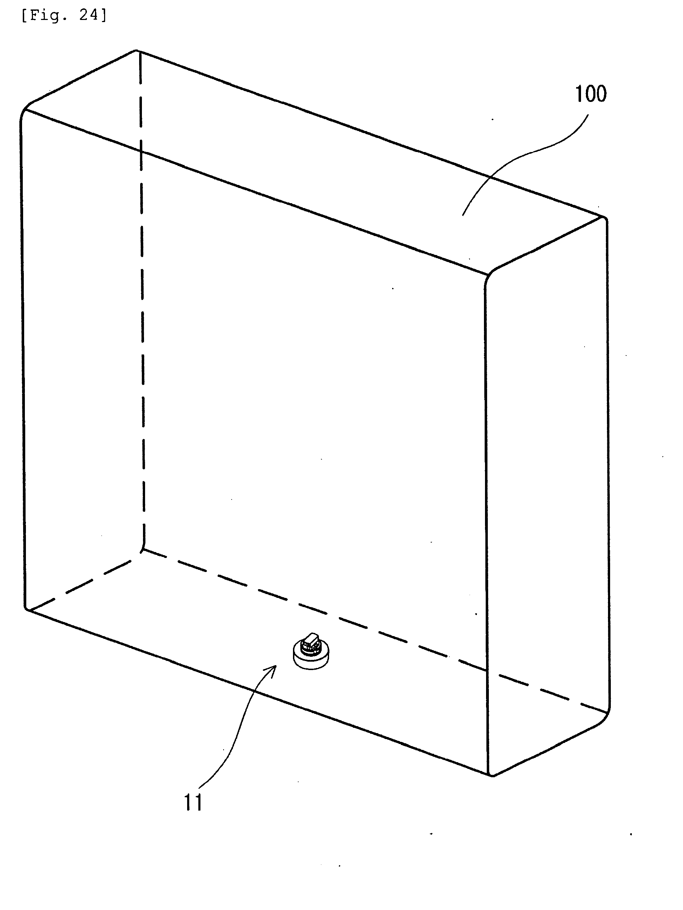

Fig. 22 is a schematic view showing a second embodiment of a fluid identification apparatus in accordance with the present invention. Fig. 23 is a schematic view showing an example of a usage state of the fluid identification apparatus of Fig. 22. Fig. 24 is a schematic view showing an example of another usage state of the fluid identification apparatus of Fig. 22.

In the configuration of a fluid identification apparatus 11 in accordance with the present embodiment of the present invention, configuration elements equivalent to those of the fluid identification apparatus 10 in accordance with the first embodiment of the present invention are numerically numbered similarly and the detailed descriptions of the equivalent elements are omitted.

In the fluid identification apparatus 11 in accordance with the present embodiment of the present invention, a fluid identification sensor module 20, a cover member 36, and a communication power connector 49 are disposed in a water proof case 16.

A power cable 40 and a communication cable 42 are connected to the communication power connector 49.

By miniaturizing the fluid identification apparatus 11 as shown in Fig. 22, the fluid identification apparatus 11 can be attached to the lower side of a fuel tank 100 as shown in Fig. 23 for instance.

As described above, the fluid identification apparatus 11 in accordance with the present embodiment of the present invention can be attached to any position of the fuel tank 100. Consequently, a position to which the fluid identification apparatus 11 is attached can be changed depending on the type and characteristics of a identified fluid that is stored into the fuel tank 100.

As described above, the identification accuracy of a identification target fluid can be improved by attaching the fluid identification apparatus 11 to the most suitable attachment position depending on the type and characteristics of a identification target fluid.

Moreover, the fluid identification apparatus 11 can be attached to the fuel tank 100 in such a manner that the fluid identification element 21 and the fluid temperature detecting element 22 of the fluid identification sensor module 20 are disposed horizontally to a fluid level as described above.

Moreover, as shown in Fig. 22, the fluid identification apparatus 11 in accordance with the present embodiment of the present invention can also be attached to the bottom face of the fuel tank 100 for instance.

In the case in which a identified fluid that is stored into the fuel tank 100 is a hydrocarbon liquid such as a light oil for instance, by attaching the fluid identification apparatus 11 to the bottom face of the fuel tank 100 as described above, the fluid identification sensor module 20 is dipped into a identification target fluid even when a fuel is consumed unless a fuel in the fuel tank 100 is emptied, whereby the identification of a identification target fluid can be carried out at any one time.

Moreover, in the case in which a identification target fluid in the fuel tank 100 is emptied, the sensor output Q is rapidly changed, whereby the existence or nonexistence of a identification target fluid can also be identified.

By attaching the fluid identification apparatus 11 to the lower side of a fuel tank 100 as shown in Fig. 23, in the case in which the existence or nonexistence of a identification target fluid is identified, a user who operates a construction machine can detect that an amount of a fuel in the fuel tank 100 is smaller than the prescribed amount for instance.

While the preferred embodiments in accordance with the present invention have been described above, the present invention is not restricted to the embodiments, and various changes, modifications, and functional additions can be thus made without departing from the scope of the present invention. For instance, a pulse voltage value, an application time of a pulse voltage, and a sampling count can be changed as needed.

The present invention is not restricted to the above described embodiments, and various changes, modifications, and functional additions can be thus made without departing from the scope of the present invention. The case in which the fluid identification apparatus is attached to a fuel tank of a construction machine or a heavy machine was described in the above embodiments. However, the fluid identification apparatus can also be applied to a gasoline tank and an oil tank that stores the lubricating oil for an automobile, and an urea tank for a decomposition of NOx for an automobile.

EXPLANATIONS OF LETTERS OR NUMERALS

10: Fluid identification apparatus
11: Fluid identification apparatus
12: support part
16: Water proof case
20: Fluid identification sensor module

21: Fluid identification element
21a: Fluid detecting thin film chip
21a1: Chip substrate
21a2: Fluid detecting temperature sensing element
21a2: Temperature sensing element
21a3: Interlayer insulation film
21a4: Electrical heating element
21a5: Electrical heating element electrode
21a6: Protective film
21a7: Electrode pad
21d: Bonding wire
21e: External electrode terminal
22: Fluid temperature detecting element
22a2: Temperature sensing element
22e: External electrode terminal
26: Mold resin
36: Cover member
38: Identified fluid introduction path
40: Power cable
42: Communication cable
49: Communication power connector
50: Control unit
54: Storage device
56: Power connection terminal
58: Interface
64: Resistor
65: Resistor
68: Bridge circuit
70: Differential amplifier
71: Fluid temperature detecting amplifier
72: Microcomputer
74: Switch
100: Fuel tank

**Claims**

1. A fluid identification apparatus for identifying a identification target fluid, comprising:

   a fluid identification sensor that includes a fluid identification element and a fluid temperature detecting element that is disposed separately at a predefined distance from the fluid identification element; and
   a identification control part that discriminates a fluid based on an output from the fluid identification sensor,

   wherein a voltage is applied to the fluid identification element for a prescribed time to heat a identification target fluid; a first output value that is an electrical output value corresponding to a first temperature of a fluid identification element and a second output value that is an electrical output value corresponding to a second temperature of a fluid identification element are obtained; and
   a fluid identification is carried out by comparing a rate of change of the first output value and the second output value with a rate of change of a first output value and a second output value for a reference fluid, which has been measured and has been stored into the identification control part.

2. The fluid identification apparatus as defined in claim 1,
   wherein the fluid identification element is provided with an electrical heating element and a temperature sensing element that is disposed close to the electrical heating element.

3. The fluid identification apparatus as defined in claim 1,
   wherein the fluid identification element is provided with a temperature sensing element that has a heat generating function and a temperature sensing function.

4. The fluid identification apparatus as defined in any preceding claim, wherein the fluid identification element and the fluid temperature detecting element are disposed horizontally to a fluid level.

5. The fluid identification apparatus as defined in any preceding claim, wherein the identification target fluid is a hydrocarbon liquid.

6. A fluid identification method for identifying a identification target fluid, comprising the steps of:

using a fluid identification sensor that includes a fluid identification element;
applying a voltage for a prescribed time to the fluid identification element to heat a identification target fluid;
obtaining a first output value that is an electrical output value corresponding to a first temperature of a fluid identification element and a second output value that is an electrical output value corresponding to a second temperature of a fluid identification element; and
carrying out a fluid identification by comparing a rate of change of the first output value and the second output value with a rate of change of a first output value and a second output value for a reference fluid that has been measured.

7. The fluid identification method as defined in claim 6,
wherein a fluid identification is carried out based on a difference between the rate of change of a first output value and a second output value for the reference fluid and the rate of change of the first output value and the second output value for the identification target fluid.

8. The fluid identification method as defined in claim 7,
wherein an output value of the fluid identification sensor is corrected in such a manner that the rate of change of a first output value and a second output value for the reference fluid is 0.

9. The fluid identification method as defined in claim 6,
wherein a fluid identification is carried out based on a ratio of the rate of change of the first output value and the second output value for the identification target fluid to the rate of change of a first output value and a second output value for the reference fluid.

10. The fluid identification method as defined in claim 9,
wherein an output value of the fluid identification sensor is corrected in such a manner that the rate of change of a first output value and a second output value for the reference fluid is 1.

11. The fluid identification method as defined in any of claims 6 to 10, wherein the first temperature is an initial temperature before a voltage is applied to the fluid identification element.

12. The fluid identification method as defined in any of claims 6 to 10, wherein a difference between the first temperature and the second temperature is at least 20°C.

13. The fluid identification method as defined in any of claims 6 to 12, wherein a identification of the identification target fluid is at least one of a fluid type identification, a concentration identification, and the fluid existence or nonexistence identification.

14. The fluid identification method as defined in any of claims 6 to 13, wherein the identification target fluid is a hydrocarbon liquid.

[Fig. 1]

[Fig. 2]

[Fig. 3]

[Fig. 4]

[Fig. 5]

[Fig. 6]

Heater control

26

Pulse voltage P

74

64

68

66

71 Fluid temperature detecting amplifier

72

70

Sensor output

Microcomputer

21

21a4

21a2

22

22a2

Output voltage Q

Output buffer circuit

76

Analog output

Digital output

[Fig. 7]

[Fig. 8]

(A)

(B)

[Fig. 9]

[Fig. 10]

[Fig. 11]

[Fig. 12]

[Fig. 13]

[Fig. 14]

[Fig. 15]

[Fig. 16]

[Fig. 17]

[Fig. 18]

[Fig. 19]

[Fig. 20]

[Fig. 21]

[Fig. 22]

[Fig. 23]

100

11

[Fig. 24]

100

11

Europäisches
Patentamt

European
Patent Office

Office européen
des brevets

**EUROPEAN SEARCH REPORT**

Application Number

EP 09 25 1800

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/187999 A1 (KAWANISHI TOSHIAKI [JP] ET AL) 24 August 2006 (2006-08-24) * abstract * * paragraphs [0082], [0094] - [0096], [0098] - [0103]; figure 6 * ----- | 1-14 | INV. G01N25/20 G01N27/18 G01N33/28 |
| Y | EP 1 752 762 A (MITSUI MINING & SMELTING CO [JP]) 14 February 2007 (2007-02-14) * paragraphs [0024], [0025], [0035] - [0037], [0045]; figure 7 * ----- | 1-4,6-13 | |
| Y | DE 39 17 935 A1 (NISSAN MOTOR [JP]) 7 December 1989 (1989-12-07) * abstract * * column 3, lines 12-45; claims 1,5 * ----- | 1-14 | |

TECHNICAL FIELDS SEARCHED (IPC)

G01N

The present search report has been drawn up for all claims

| Place of search | Date of completion of the search | Examiner |
|---|---|---|
| Munich | 22 September 2009 | Filipas, Alin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

......................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 25 1800

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2009

| Patent document cited in search report | | Publication date | Patent family member(s) | | Publication date |
|---|---|---|---|---|---|
| US 2006187999 | A1 | 24-08-2006 | CA | 2532208 A1 | 20-01-2005 |
| | | | EP | 1645870 A1 | 12-04-2006 |
| | | | JP | 2005030887 A | 03-02-2005 |
| | | | WO | 2005005970 A1 | 20-01-2005 |
| EP 1752762 | A | 14-02-2007 | JP | 4038492 B2 | 23-01-2008 |
| | | | JP | 2005337969 A | 08-12-2005 |
| | | | WO | 2005116620 A1 | 08-12-2005 |
| | | | US | 2008066531 A1 | 20-03-2008 |
| DE 3917935 | A1 | 07-12-1989 | JP | 1304348 A | 07-12-1989 |

EPO FORM P0459

For more details about this annex : see Official Journal of the European Patent Office, No. 12/82